(19) Europäisches Patentamt European Patent Office Office européen des brevets

(11) **EP 4 266 281 A1**

(12) **DEMANDE DE BREVET EUROPEEN**

(43) Date de publication:
**25.10.2023 Bulletin 2023/43**

(21) Numéro de dépôt: **23168960.5**

(22) Date de dépôt: **20.04.2023**

(51) Classification Internationale des Brevets (IPC):
**G08B 21/06** (2006.01)   **A61B 5/00** (2006.01)
**B60K 28/00** (2006.01)

(52) Classification Coopérative des Brevets (CPC):
**G08B 21/06; A61B 5/00; G06F 18/00;**
G08B 21/0446; G08B 21/0453; G08B 21/0476;
G08B 29/186; G08B 29/188

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(30) Priorité: **22.04.2022 FR 2203751**

(71) Demandeurs:
• **THALES**
**92400 Courbevoie (FR)**
• **UNIVERSITE DE BORDEAUX**
**33000 Bordeaux (FR)**
• **Institut Polytechnique de Bordeaux**
**33400 Talence (FR)**
• **Centre national de la recherche scientifique**
**75016 Paris (FR)**

(72) Inventeurs:
• **BERTHELOT, Bastien**
**33700 Merignac (FR)**
• **IBANEZ, Vincent**
**33700 Merignac (FR)**
• **BECOUARN, Loïc**
**33700 Merignac (FR)**
• **MAZOYER, Patrick**
**33700 Merignac (FR)**
• **LEGRAND, Pierrick**
**33405 Talence (FR)**
• **GRIVEL, Eric**
**33405 Talence (FR)**

(74) Mandataire: **Lavoix**
**2, place d'Estienne d'Orves**
**75441 Paris Cedex 09 (FR)**

(54) **DISPOSITIF ÉLECTRONIQUE DE SURVEILLANCE D'UN ÉTAT DE CONSCIENCE D'UN OPÉRATEUR DANS UN AÉRONEF, PROCÉDÉ ET PROGRAMME D'ORDINATEUR ASSOCIÉS**

(57) L'invention concerne un dispositif électronique de surveillance (22) d'un état de conscience d'un opérateur (14) dans un poste de commande (16) d'un aéronef (12).

Le dispositif de surveillance (22) comprend :
- un module de réception d'une donnée d'au moins deux capteurs embarqués dans l'aéronef (12), au moins l'un des capteurs dit capteur porté (24) étant au contact physique dudit opérateur (14) et au moins l'un des capteurs dit capteur déporté (26) étant à distance dudit opérateur (14) ;
- un module de traitement configuré pour extraire de chaque donnée au moins un paramètre représentatif de l'état de conscience de l'opérateur (14) ;
- un module de fusion configuré pour recevoir les dits paramètres représentatifs et mettre en oeuvre une méthode d'apprentissage automatique pour déterminer en fonction des paramètres représentatifs si l'opérateur (14) est dans un état de conscience nominal ou altéré.

FIG.2

**Description**

La présente invention concerne un dispositif électronique de surveillance d'un état de conscience d'un opérateur dans un aéronef.

L'invention concerne également un procédé de commande de surveillance d'un état de conscience d'un opérateur dans un aéronef.

L'invention concerne également un programme d'ordinateur comportant des instructions logicielles qui, lorsqu'elles sont exécutées par un ordinateur, mettent en oeuvre un tel procédé.

L'aéronef est typiquement un avion, un hélicoptère, ou encore un drone. L'opérateur est par exemple le pilote de l'aéronef, le copilote ou un opérateur à distance (i.e. pilote de drone ou opérateur radar). Le poste de commande est notamment disposé dans l'aéronef ou est disposé à distance de l'aéronef dans le cas d'un drone par exemple.

La surveillance de l'état de conscience d'un tel opérateur est indispensable pour la sécurité de l'aéronef afin de détecter une perte de connaissance qui peut entrainer une incapacité de l'opérateur à réaliser les taches prévues dans des conditions opérationnelles de vol.

De manière conventionnelle, l'état de conscience d'un opérateur est surveillé par les autres opérateurs de l'aéronef. Par exemple le co-pilote surveille l'état de conscience du pilote, et inversement. Pour compléter cette surveillance, il a été proposé une surveillance au moyen d'un capteur disposé dans l'aéronef.

Toutefois, une telle méthode ne donne pas entière satisfaction car elle manque de réactivité. En effet, il est nécessaire de traiter les données issues du capteur sur une fenêtre d'analyse temporelle amenant un effet de tampon (ou « buffering » en anglais) en cas de perte de signal temporaire. Il est alors nécessaire d'attendre la durée de la fenêtre d'analyse, généralement plusieurs secondes, avant d'avoir à nouveau une information valide relative à l'état de conscience du pilote. On conçoit ainsi que la réactivité d'une telle méthode n'est pas suffisante en cas de perte de conscience du pilote par exemple et où il est important de réagir vite, parfois en moins d'une seconde.

En outre, ce type de méthode entraîne un grand nombre de faux-positifs, notamment lorsque des turbulences ou vibrations perturbent le signal issu du capteur. Il est essentiel de limiter les faux-positifs car les contremesures qu'ils impliquent impactent fortement le fonctionnement de l'aéronef: émission intempestive d'alertes, reprise en main automatique de l'aéronef, intervention extérieure, etc.

De plus, les méthodes connues ne sont pas assez robustes à la variabilité des situations et au nombre limité de données. En particulier, il est nécessaire que la méthode de surveillance soit robuste aux variabilités des situations rencontrées. En effet, la détection d'une perte de conscience doit être robuste à la variabilité des caractéristiques physiologiques des différents opérateurs (âge, genre, etc.), mais aussi à la variabilité de l'environnement dans lequel se trouve l'opérateur (cockpit d'avion en turbulence, cockpit où la luminosité ambiante varie, etc.). Enfin, la méthode doit être robuste malgré le peu de données opérationnelles à disposition correspondant aux comportements recherchés. Dans le domaine militaire, ce problème est d'autant plus complexe que les données collectées et utiles à l'application d'intérêt ne sont pas nécessairement disponibles puisque souvent classifiées, et peu nombreuses en raison de la difficulté d'observation de situations critiques.

Il existe donc un besoin pour un dispositif de surveillance de l'état de conscience d'un opérateur permettant une meilleure réactivité tout en étant précis, fiable et robuste.

A cet effet, l'invention a pour objet un dispositif électronique de surveillance d'un état de conscience d'un opérateur dans un poste de commande d'un aéronef, le dispositif de surveillance comprenant :

- un module de réception configuré pour recevoir une donnée d'au moins deux capteurs embarqués dans l'aéronef, au moins l'un des capteurs dit capteur porté étant au contact physique dudit opérateur et au moins l'un des capteurs dit capteur déporté étant à distance dudit opérateur ;
- un module de traitement configuré pour extraire de chaque donnée au moins un paramètre représentatif de l'état de conscience de l'opérateur ;
- un module de fusion configuré pour recevoir les dits paramètres représentatifs et mettre en oeuvre une méthode d'apprentissage automatique pour déterminer en fonction des paramètres représentatifs si l'opérateur est dans un état de conscience nominal ou dans un état de conscience altéré.

Ainsi, la présente invention repose sur l'utilisation de plusieurs capteurs de différents types, portés ou déportés, et la fusion des paramètres représentatifs obtenus au moyen des différents traitements des données issues des capteurs. La surveillance est ainsi plus robuste aux variabilités dues à l'opérateur, à l'environnement ainsi qu'aux conditions de vol, tout en conservant une réactivité accrue à la détection d'un état de conscience dégradé de l'opérateur. En effet, multiplier et diversifier les canaux de collecte de données via les différents capteurs assure une meilleure disponibilité de la détection et permet de réduire le nombre de faux-positifs.

Suivant d'autres aspects avantageux de l'invention, le dispositif électronique de surveillance comprend une ou plusieurs des caractéristiques suivantes, prises isolément ou suivant toutes les combinaisons techniquement possibles :

- le dispositif de surveillance comprend en outre un module d'alerte configuré pour émettre un signal d'alerte lorsque le module de fusion détermine que l'opérateur est dans un état de conscience altéré ;

- chaque capteur porté est choisi parmi le groupe constitué de :

  + un capteur cardiaque, notamment un électrocardiographe ;
  + un oxymètre de pouls, notamment un capteur à photopléthysmographie ;
  + un capteur de respiration ;
  + un accéléromètre ;
  + une électrode crânienne, par exemple un électroencéphalographe ;
  + un capteur de pression agencé dans un siège de l'opérateur ;
  + un capteur de pression agencé dans un dispositif de commande propre à être actionné par l'opérateur ;
  + un capteur de sudation de l'opérateur ;
  + un capteur de réponse électrodermale ;
  + un capteur de température interne de l'opérateur;
  + un bandeau à spectroscopie proche infrarouge ;

- au moins l'un des capteurs portés est un capteur de pression configuré pour mesurer au moins une pression appliquée par l'opérateur sur le capteur de pression, le paramètre associé propre à être extrait par le module de traitement étant une durée durant laquelle la pression mesurée est supérieure à une pression seuil prédéterminée ;

- au moins l'un des capteurs portés est un accéléromètre configuré pour mesurer une accélération d'au moins une partie de l'opérateur, le paramètre associé propre à être extrait par le module de traitement étant une signature issue d'une analyse fréquentielle et/ou une analyse temporelle de l'accélération mesurée et choisi parmi le groupe constitué de :

  + une puissance portée par une bande de fréquence de l'accélération mesurée ;
  + un rapport entre puissances de bandes de fréquences de l'accélération mesurée ;
  + la puissance de l'accélération mesurée ;
  + la moyenne de l'accélération mesurée ;
  + le taux de passage par zéro de l'accélération mesurée ;
  + la régularité de l'accélération mesurée ;
  + la complexité de l'accélération mesurée ;
  + l'entropie de l'accélération mesurée ;
  + les paramètres d'une modélisation de l'accélération mesurée ;
  + les coefficients issus d'une analyse temps fréquence de l'accélération mesurée ;
  + les coefficients issus d'une analyse temps échelle de l'accélération mesurée ;

- chaque capteur déporté est choisi parmi le groupe constitué de :

  + une caméra configurée pour capter au moins une image comprenant au moins une partie de l'opérateur ;
  + un microphone pour capter au moins une son émis par l'opérateur, tel que sa voix ou sa respiration ;
  + un capteur infrarouge de température de peau de l'opérateur ;

- au moins l'un des capteurs déportés est une caméra configurée pour capter au moins une image comprenant au moins une partie de l'opérateur, chaque paramètre propre à être extrait par le module de traitement étant choisi parmi le groupe constitué de :

  + un mouvement de l'opérateur ;
  + une posture de l'opérateur ;
  + une orientation de la tête de l'opérateur ;
  + une direction du regard de l'opérateur ;
  + une ouverture partielle des yeux de l'opérateur;
  + un clignement des yeux de l'opérateur;
  + une information de structure de l'image dans laquelle l'opérateur apparaît ;

- au moins l'un des capteurs portés est un capteur de pression, au moins l'un des capteurs portés est un accéléromètre et au moins l'un des capteurs déporté est une caméra ;

- le module de traitement est configuré pour extraire de chaque donnée au moins un paramètre représentatif de l'état de conscience de l'opérateur en mettant en oeuvre pour chaque donnée un algorithme choisi parmi le groupe constitué de:

  + une extraction d'une caractéristique prédéterminée de la donnée associée suivi d'une méthode d'apprentissage automatique ;
  + une méthode d'apprentissage profond appliquée directement sur la donnée associée ;
  + une modélisation prédéterminée appliquée à la donnée associée.

L'invention concerne également un procédé de surveillance d'un état de conscience d'un opérateur dans un poste de commande d'un aéronef, le procédé de surveillance comprenant au moins les étapes suivantes :

- réception d'une donnée d'au moins deux capteurs embarqués dans l'aéronef, au moins l'un des capteurs dit capteur porté étant au contact physique dudit opérateur et au moins l'un des capteurs dit capteur déporté étant à distance dudit opérateur ;

- extraction de chaque donnée d'au moins un paramètre représentatif de l'état de conscience de l'opérateur ;

- réception des dits paramètres représentatifs et mise en oeuvre d'une méthode d'apprentissage automatique pour déterminer en fonction des paramètres représentatifs si l'opérateur est dans un état de conscience nominal ou dans un état de conscience altéré.

L'invention a également pour objet un programme d'ordinateur comportant des instructions logicielles qui, lorsqu'elles sont exécutées par un ordinateur, mettent en oeuvre un procédé de surveillance tel que défini ci-dessus.

Ces caractéristiques et avantages de l'invention apparaîtront plus clairement à la lecture de la description qui va suivre, donnée uniquement à titre d'exemple non limitatif, et faite en référence aux dessins annexés, sur lesquels :

    **[Fig 1]** la figure 1 est une représentation schématique d'un aéronef comprenant un dispositif de surveillance selon l'invention ;
    **[Fig 2]** la figure 2 est une représentation schématique d'un poste de commande dans l'aéronef de la figure 1, et
    **[Fig 3]** la figure 3 est un organigramme d'un procédé de surveillance selon l'invention mis en oeuvre par le dispositif électronique.

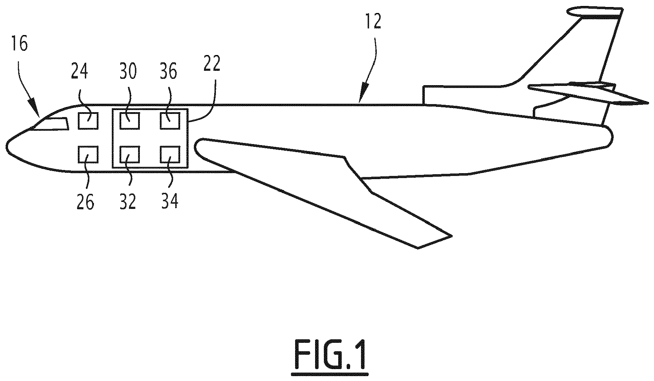

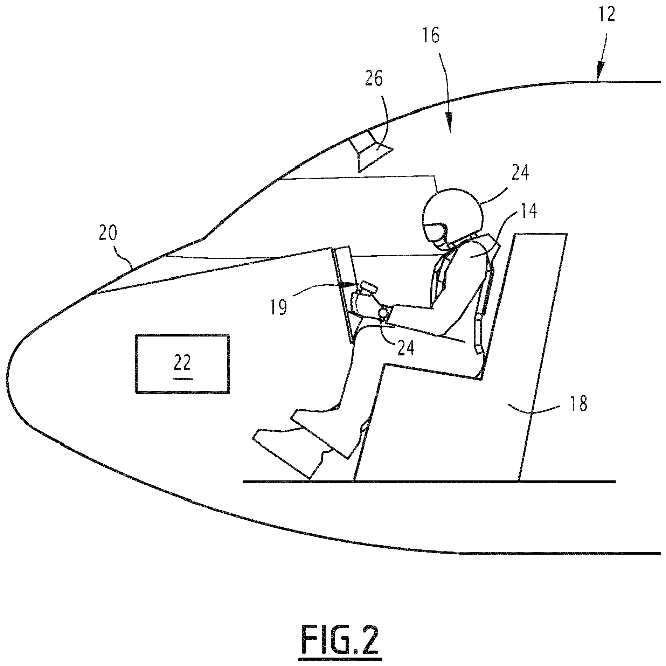

Un aéronef 12 est représenté sur la figure 1.
L'aéronef 12 est typiquement un avion, un hélicoptère, ou encore un drone. Autrement dit, l'aéronef 12 est un engin volant pilotable par un opérateur 14 via un poste de commande 16. Le poste de commande 16 est disposé à l'intérieur de l'aéronef 12 ou bien à distance de l'aéronef 12, notamment dans le cas d'un drone.
L'opérateur 14 est ici un pilote mais l'invention s'applique de manière similaire à tout opérateur de l'aéronef 12 tel qu'un co-pilote ou un opérateur radar.
Comme visible sur la figure 2, le poste de commande 16 est ici un cockpit de l'aéronef 12. Comme visible sur la figure 1, le poste de commande 16 comporte au moins un siège 18 pour l'opérateur 14, un dispositif de commande 19 propre à être actionné par l'opérateur 14, un pare-brise 20 au moins partiellement transparent et séparant l'intérieur du cockpit de l'environnement extérieur de l'aéronef 12, une pluralité de capteurs et un dispositif électronique de surveillance 22 d'un état de conscience de l'opérateur 14.
Chaque capteur est configuré pour mesurer au moins une information relative à l'opérateur 14 et notamment à son état de conscience, comme cela sera expliqué plus en détail par la suite.
Le poste de commande 16 comprend au moins deux capteurs.
Au moins l'un des capteurs est un capteur porté 24 et au moins l'un des capteurs est un capteur déporté 26.
Avantageusement, deux des capteurs sont des capteurs portés 24 et un des capteurs est un capteur déporté 26.
Un capteur porté 24 est un capteur propre à être au contact physique de l'opérateur 14. L'homme du métier comprendra qu'on entend par « au contact » que le capteur 24 touche une partie de l'opérateur, avec éventuellement un habit entre le capteur et la peau de l'opérateur 14. Un capteur porté 24 n'est donc pas nécessairement un accessoire porté en permanence par l'opérateur 14 comme une montre au poignet, le capteur porté 24 est éventuellement au contact de l'opérateur 14 de manière discontinue, comme par exemple un capteur de pression disposé sur un dispositif de commande 19. Ainsi, le capteur porté 24 se présente par exemple sous la forme d'une montre au poignet de l'opérateur, d'un casque sur la tête de l'opérateur 14 ou d'un capteur intégré dans le dispositif de commande 19 ou dans le siège 18.
En particulier, chaque capteur porté 24 est choisi parmi le groupe constitué de :

- un capteur cardiaque, notamment un électrocardiographe ;
- un oxymètre de pouls, notamment un capteur à photopléthysmographie ;
- un capteur de respiration ;
- un accéléromètre ;
- une électrode crânienne, par exemple un électroencéphalographe ;
- un capteur de pression agencé dans un siège 18 de l'opérateur 14 ;
- un capteur de pression agencé dans un dispositif de commande 19 propre à être actionné par l'opérateur 14 ;
- un capteur de sudation de l'opérateur 14 ;
- un capteur de réponse électrodermale ;
- un capteur de température interne de l'opérateur;
- un bandeau à spectroscopie proche infrarouge.

Un capteur déporté 26 est un capteur arrangé à distance de l'opérateur 14 lorsque la mesure du capteur déporté 26 est faite, lors des conditions opérationnelles de vol. L'homme du métier comprendra qu'on entend par « à distance », qu'il existe un espace vide entre le capteur 26 et l'opérateur 14 lors de la mesure par le capteur déporté 26.
En particulier, chaque capteur déporté 26 est choisi parmi le groupe constitué de :

- une caméra configurée pour capter au moins une image comprenant au moins une partie de l'opérateur 14 ;
- un microphone pour capter au moins une son émis par l'opérateur 14, tel que sa voix ou sa respiration ;
- un capteur infrarouge de température de peau de l'opérateur 14.

Dans un mode de réalisation avantageux, au moins l'un des capteurs portés 24 est un capteur de pression, au moins l'un des capteurs portés 24 est un capteur de pression, au moins l'un des capteurs portés 24 est un accéléromètre et au moins l'un des capteurs déporté 26 est une caméra. Ainsi, trois types de capteurs sont présents dans le poste de commande 16 permettant de multiplier et diversifier les canaux de collecte de données. Avantageusement, une pluralité de capteurs de chaque type sont présents dans le poste de commande 16, par exemple deux caméras, six capteurs de pression et quatre accéléromètres.

Le dispositif électronique de surveillance 22 est configuré pour surveiller un état de conscience de l'opérateur 14. L'état de conscience est représentatif de la capacité de l'opérateur 14 à prendre connaissance de son propre état et de son environnement afin de réagir en fonction.

En particulier, l'état de conscience peut être un état de conscience dit « nominal », correspondant à l'état de conscience attendu de l'opérateur 14 pendant un vol d'un aéronef 12, c'est-à-dire un état éveillé et lucide.

L'état de conscience peut être un état de conscience dit « altéré », correspondant à un état de conscience de perte au moins partielle de conscience du monde extérieur par l'opérateur 14, comme par exemple un état de somnolence, de sommeil ou d'évanouissement. Dans cet état de conscience, l'opérateur a une connaissance altérée voire inexistante de son environnement et ne peut réagir en fonction. Cet état de conscience altéré est problématique lors du vol de l'aéronef 12 car l'opérateur 14 n'est pas apte à réaliser les tâches qu'il a à accomplir de manière réactive et pertinente.

A cet effet, le dispositif de surveillance 22 est configuré pour déterminer si l'opérateur 14 est dans un état de conscience nominal ou dans un état de conscience altéré.

En particulier, le dispositif de surveillance 22 comprend un module de réception 30, un module de traitement 32 et un module de fusion 34.

Le dispositif de surveillance 22 comprend avantageusement en outre un module d'alerte 36.

Le module de réception 30 est configuré pour recevoir une donnée d'au moins deux capteurs embarqués dans l'aéronef 12, dont au moins un capteur porté 24 et au moins un capteur déporté 26.

Le module de traitement 32 est configuré pour extraire de chaque donnée reçue par le module de réception 30 au moins un paramètre représentatif de l'état de conscience de l'opérateur 14.

Un paramètre représentatif de l'état de conscience est un paramètre défini par exemple par des experts du domaine et permettant d'avoir une information sur l'état de conscience du pilote. Par exemple, un rythme de fréquence cardiaque bas, des yeux fermés sur une longue période de temps, une pression constante exercée, une position de la tête inclinée etc., sont des paramètres permettant de déterminer que l'opérateur est dans un état de conscience altéré.

En particulier, lorsqu'au moins l'un des capteurs portés 24 est un capteur de pression configuré pour mesurer au moins une pression appliquée par l'opérateur 14 sur le capteur de pression, le paramètre associé propre à être extrait par le module de traitement 32 est une durée durant laquelle la pression mesurée est supérieure à une pression seuil prédéterminée. En effet, une telle situation traduit une perte de connaissance de l'opérateur 14 qui exerce une pression importante en continu sur une partie du siège 18 ou sur le dispositif de commande 19.

En variante ou en complément, lorsqu'au moins l'un des capteurs portés 24 est un accéléromètre configuré pour mesurer une accélération d'au moins une partie de l'opérateur 14, le paramètre associé propre à être extrait par le module de traitement 32 est une signature issue d'une analyse fréquentielle et/ou une analyse temporelle de l'accélération mesurée et choisi parmi le groupe constitué de :

- une puissance portée par une bande de fréquence de l'accélération mesurée ;
- un rapport entre puissances de bandes de fréquences de l'accélération mesurée;
- la puissance de l'accélération mesurée ;
- la moyenne de l'accélération mesurée ;
- le taux de passage par zéro de l'accélération mesurée ;
- la régularité de l'accélération mesurée ;
- la complexité de l'accélération mesurée ;
- l'entropie de l'accélération mesurée ;
- les paramètres d'une modélisation a priori de l'accélération mesurée ;
- les coefficients issus d'une analyse temps fréquence de l'accélération mesurée ;
- les coefficients issus d'une analyse temps échelle de l'accélération mesurée.

Concernant l'analyse fréquentielle, le module de traitement 32 est configuré pour estimer la puissance portée par des bandes de fréquence, ou des rapports de puissance pertinents.

Concernant l'analyse temporelle, le module de traitement 32 est configuré pour déterminer un paramètre tel que la moyenne, la puissance ou le taux de passage par zéro du signal, la régularité, la complexité et l'entropie du signal. Différents marqueurs peuvent être considérés pour cela, tels que l'entropie multi-échelle ou le coefficient de Hurst qui sont détaillés ci-dessous.

Le terme Entropie est utilisé dans de nombreux domaines, de la thermodynamique à la théorie de l'information en passant par la mécanique statistique et la structure des graphes.

En théorie de l'information, l'entropie de Shannon est l'une des entropies les plus populaires depuis plus de 70 ans, mais d'autres entropies ont été développées en particulier dans les années 60 et 70 avec de nombreux contributeurs tels qu'Arimoto et Picard lorsqu'ils traitent de variables aléatoires discrètes. Certaines généralisations de l'entropie de Shannon ont été proposées comme l'entropie de Sharma-Mittal qui dépendent de deux paramètres réels : $\alpha \neq 1$ que l'on nomme l'ordre et β≠ 1 qui est le degré. Lorsque α= β, on obtient l'entropie de Tsallis tandis que lorsque β tend vers 1, cela conduit à l'entropie Rényi pour α>0. Dans ce dernier cas, lorsque α tend vers 1, on retrouve l'entropie de Shannon. Il est à noter que l'on aboutit à l'entropie de Hartley (resp. min entropie) quand α tend vers 0 (resp. +∞). Enfin, le cas α=2 correspond à l'entropie de collision.

La notion d'entropie d'un système dynamique à F degrés de liberté a été introduite à la fin des années 50. Cela a conduit à l'entropie de Kolmogorov-Sinai (KS) dont la valeur permet de distinguer un système ordonné d'un système chaotique. Ce fut le point de départ de nouveaux travaux de recherche dont le but était d'obtenir une approximation de l'entropie KS en pratique. Il en a résulté des grandeurs telles que l'entropie $K\_2$ proposée par Grassberger et Procaccia, l'entropie d'Eckmann-Ruelle et enfin l'entropie approximative (ApEn) proposée par Pincus en 1991 [Pincus1991].

La « Sample entropy » (SampEn) est une extension de l'ApEn ; elle fut proposée par Richman et Moorman il y a une vingtaine d'années [Richman2000]. Dans [Jiang2011], l'expression théorique de la SampEn pour un bruit blanc est donnée. Bien que très utilisée, la SampEn est sensible aux signaux de courte durée. Des variantes ont été développées comme le coefficient d'entropie de l'échantillon (COSEn) proposé par Lake et Moorman pour résoudre le problème de la fibrillation auriculaire. Il correspond à la SampEn à laquelle on a retranché des termes comme le logarithme de l'intervalle RR (temps entre deux battements cardiaques) [Lake2011]. Chen et al. [Chen2007] [Chen2009] ont suggéré d'utiliser le concept d'ensembles flous de Zadeh dans la procédure de classification de l'ApEn et de la SampEn, ce qui a donné lieu à l'entropie floue (FuzzyEn). Il y a aussi l'entropie dite de distribution [Rostaghi2016], l'entropie de permutation [Bandt2002] et l'entropie hiérarchique [Jiang2011]. Le lecteur peut se référer aux articles pour plus de détails.

Effectuer une classification de séries temporelles telles que l'évolution des intervalles inter-battements n'est pas nécessairement une tâche facile si une seule « échelle » du signal est considérée. Par conséquent, la prise en compte de différentes échelles du signal peut être envisagée. Cela conduit aux entropies dites multi-échelles.

Il y a vingt ans, l'entropie multi-échelle (MSE) a été proposée par Costa [Costa2002] pour évaluer la complexité d'un signal. Elle consiste à sommer la SampEn du signal lui-même mais aussi les SampEn de séries temporelles correspondant à ce que l'on appelle une nouvelle « échelle » $\tau$ du signal : ces séries se déduisent comme suit : 1/ effectuer sur le signal d'origine un filtrage moyenneur, passe-bas de fréquence de coupure de plus en plus basse quand $\tau$ augmente 2/ décimer le signal filtré d'un facteur $\tau$.

En 2009, la refined multiscale entropy est proposée par Valencia et al. [Valencia 2009] où le filtre à réponse impulsionnelle finie passe-bas est remplacé par un filtre passe-bas à réponse impulsionnelle infinie dont la fréquence de coupure est choisie pour sous-échantillonner correctement le signal (c'est-à-dire éviter les problèmes de recouvrement de spectre quand la fréquence d'échantillonnage diminue), ce qui n'était pas le cas dans la MSE standard. Cependant, le filtre proposé n'est pas à phase linéaire et introduit une distorsion de phase dans la bande passante. En 2013, la composite multiscale entropy proposée par Wu et al. consiste à combiner les $\tau$ séquences qui peuvent être définies après l'étape de décimation, alors que la MSE ne conserve que la première. La refined composite multiscale entropy fut ensuite mise en oeuvre.

Tirant avantage des entropies alternatives à la SampEn, d'autres entropies multi-échelles ont été proposées ces dernières années. Parmi elles et sans être exhaustif dans ce document, on peut citer l'entropie de permutation multi-échelle [Vakharia2015], l'entropie d'échantillon floue multi-échelle (MFE), l'entropie floue composite multi-échelle, composite et raffinée dans [Zheng2017], l'entropie de permutation multi-échelles composite raffinée [Humeau-Heurtier] et l'entropie multi-échelle généralisée [Costa2015].

L'analyse de l'exposant de Hurst d'un signal va désormais être expliquée. Cette analyse peut se faire par différentes méthodes. L'état de l'art se ramène à deux grandes familles : les estimateurs reposant sur l'analyse fréquentielle du signal et les estimateurs reposant sur l'analyse temporelle du signal. Il existe aussi des méthodes fondées sur des algorithmes évolutionnaires.

Afin d'estimer le coefficient de Hurst d'un processus mono-fractal, la méthode nommée « Fluctuation Analysis » (FA) (ou analyse fluctuante en français) peut être mise en oeuvre. Le principe est le suivant : après intégration du signal conduisant à une nouvelle séquence $y_{int}$, on calcule pour différentes valeurs de $l$ la grandeur suivante : $F(N) = \sqrt{< \left( y_{int}(i+N) - y_{int}(i) \right)^2 >}$

, où $<.>$ représente la moyenne temporelle. Puisque $F(N) \propto N^H$, où $\propto$ représente une relation de proportionnalité, $\log(F(N))$ est alors représenté en fonction de $\log(N)$ afin d'estimer la valeur de $H$ : celle-ci est égale à la valeur de la pente de la droite de régression.

Il est possible d'utiliser le DFA (de l'anglais « Detrended Fluctuation Analysis » ou « Analyse de la fluctuation tendue » en français ) ou le DMA (de l'anglais « Detrended Moving Average Analysis » ou « Analyse de la moyenne mobile tendue » en français) et leurs variantes pour estimer $H$. Elles reposent sur un même principe : l'analyse des fluctuations autour d'une tendance du signal centré et intégré. L'analyse est alors menée sur un processus appelé le résidu, défini comme la différence entre la version intégrée du signal et la tendance.

En variante ou en complément, lorsqu'au moins l'un des capteurs est une caméra configurée pour capter au moins une image comprenant au moins une partie de l'opérateur 14, chaque paramètre propre à être extrait par le module de traitement 32 est choisi parmi le groupe constitué de :

- un mouvement de l'opérateur 14;
- une posture de l'opérateur 14;
- une orientation de la tête de l'opérateur 14;
- une direction du regard de l'opérateur 14;
- une ouverture partielle des yeux de l'opérateur 14;
- un clignement des yeux de l'opérateur 14 ;
- une information de structure de l'image dans laquelle l'opérateur 14 apparaît.

Une information de structure de l'image dans laquelle l'opérateur 14 apparaît est par exemple une analyse de la répartition des couleurs des pixels de l'image. Un état nominal est associé à une répartition nominale avec par exemple des niveaux de bleu associés au ciel, des niveaux de gris associés à la cabine de l'aéronef, des niveaux de beige associé à l'opérateur 14 ; etc. Une répartition différente de cette répartition nominale peut être le signe d'un état de conscience altéré de l'opérateur 14. Par exemple, une image toute grise peut être le signe d'un opérateur inconscient obstruant avec son corps la caméra. Une telle méthode ne nécessite donc pas de passer par une étape de détection des formes présentes dans l'image et présente une disponibilité et une rapidité importante.

Avantageusement, le module de traitement 32 est configuré pour extraire de chaque donnée au moins un paramètre représentatif de l'état de conscience de l'opérateur 14 en effectuant une extraction d'une caractéristique prédéterminée de la donnée associée suivi d'une méthode d'apprentissage automatique.

A titre d'exemple, la caractéristique est la position de la tête de l'opérateur 14 extraite d'une vidéo obtenue par une caméra. Puis une méthode d'apprentissage automatique est mise en oeuvre pour traiter la position de la tête au cours du temps et en déduire l'état de conscience de l'opérateur 14.

Une méthode d'apprentissage automatique (ou « Machine Learning » en anglais) permet d'obtenir un modèle capable de résoudre des tâches sans être explicitement programmé pour chacune de ces tâches. L'apprentissage automatique comporte deux phases. La première phase consiste à définir un modèle à partir de données présentes dans une base de données d'apprentissage, appelées aussi observations. La définition du modèle consiste notamment ici à l'entraîner à reconnaître une perte de connaissance. Cette phase dite d'apprentissage est généralement réalisée préalablement à l'utilisation pratique du modèle. La seconde phase correspond à l'utilisation du modèle : le modèle étant défini, de nouvelles données peuvent alors être soumises au modèle afin de déterminer l'état de conscience de l'opérateur 14.

En variante, le module de traitement 32 est configuré pour extraire de chaque donnée au moins un paramètre représentatif de l'état de conscience de l'opérateur 14 en mettant en oeuvre une méthode d'apprentissage profond appliquée directement sur la donnée associée.

Une méthode d'apprentissage profond (ou « Deep Learning ») est une technique de reposant sur le modèle des réseaux neurones: des dizaines voire des centaines de couches de neurones sont empilées pour apporter une plus grande complexité au modèle. En particulier, un réseau de neurones est en général composé d'une succession de couches dont chacune prend ses entrées sur les sorties de la précédente. Chaque couche est composée d'une pluralité de neurones, prenant leurs entrées sur les neurones de la couche précédente. À chaque synapse entre neurones est associé un poids synaptique, de sorte que les entrées reçues par un neurone sont multipliées par ce poids, puis additionnées par ledit neurone. Le réseau de neurones est optimisé grâce aux ajustements des différents poids synaptiques pendant son entrainement en fonction des données présentes dans la base de données d'apprentissage. Le réseau de neurones ainsi optimisé est alors le modèle. Un nouveau jeu de données peut alors être donné en entrée du réseau de neurones qui fournit alors le résultat de la tâche pour laquelle il a été entrainé.

En variante, le module de traitement 32 est configuré pour extraire de chaque donnée au moins un paramètre représentatif de l'état de conscience de l'opérateur 14 en mettant en oeuvre une modélisation prédéterminée appliquée à la donnée associée.

La modélisation prédéterminée est par un exemple un modèle physique comprenant un ensemble de règles prédéterminées par un expert.

Le module de fusion 34 est configuré pour recevoir les dits paramètres représentatifs et mettre en oeuvre une méthode d'apprentissage automatique pour déterminer en fonction des paramètres représentatifs si l'opérateur est dans un état de conscience nominal ou dans un état de conscience altéré.

La méthode d'apprentissage automatique utilisée par le module de fusion 34 est éventuellement différente de celle utilisée par le module de traitement 32.

La méthode d'apprentissage automatique est entrainée en amont des phases opérationnelles de vol en étudiant quels paramètres sont significatifs et pertinents pour caractériser l'état de conscience de l'opérateur 14, par exemple avec l'aide d'experts. Des données expérimentales ou un retour d'expérience de vols passés peut être utilisé.

Le module d'alerte 36 est configuré pour émettre un signal d'alerte lorsque le module de fusion 34 détermine que l'opérateur est dans un état de conscience altéré.

Le signal d'alerte est par exemple un signal sonore émis dans le poste de commande 16 permettant de faire revenir l'opérateur 14 à un état de conscience nominal.

En variante ou en complément, le signal d'alerte est par exemple un signal envoyé à un système de contrôle de l'aéronef 12 pour passer en mode automatique et qu'ainsi les taches à réaliser par l'opérateur 14 soient réalisées de manière autonome sans l'intervention de l'opérateur 14. Notamment, lorsque l'opérateur 14 est un pilote, l'aéronef 10 passe en pilote automatique.

En variante ou en complément, le signal d'alerte est par exemple un signal de communication vers un dispositif de contrôle externe à l'aéronef 12 tel qu'une tour de contrôle.

Dans l'exemple de la figure 1, le dispositif électronique de surveillance 22 comprend une unité de traitement d'informations formée par exemple d'une mémoire et d'un processeur associé à la mémoire. Le module de réception 30, le module de traitement 32, le module de fusion 34, et en complément facultatif, le module d'alerte 36 sont réalisés chacun sous forme d'un logiciel, ou d'une brique logicielle, exécutables par le processeur. La mémoire est alors apte à stocker un logiciel de réception, un logiciel de traitement, un logiciel de fusion, et en complément facultatif, un logiciel d'alerte. Le processeur est alors apte à exécuter chacun de ces logiciels.

En variante non représentée, module de réception 30, le module de traitement 32, le module de fusion 34, et en complément facultatif, le module d'alerte 36 sont réalisés chacun sous forme d'un composant logique programmable, tel qu'un FPGA (de l'anglais Field Programmable Gate Array), ou encore sous forme d'un circuit intégré dédié, tel qu'un ASIC (de l'anglais Application Spécifie Integrated Circuit).

Lorsque le dispositif électronique 22 est réalisé sous forme d'un ou plusieurs logiciels, c'est-à-dire sous forme d'un programme d'ordinateur, il est en outre apte à être enregistré sur un support, non représenté, lisible par ordinateur. Le support lisible par ordinateur est par exemple, un médium apte à mémoriser les instructions électroniques et à être couplé à un bus d'un système informatique. A titre d'exemple, le support lisible est un disque optique, un disque magnéto-optique, une mémoire ROM, une mémoire RAM, tout type de mémoire non-volatile (par exemple EPROM, EEPROM, FLASH, NVRAM), une carte magnétique ou une carte optique. Sur le support lisible est alors mémorisé un programme d'ordinateur comportant des instructions logicielles.

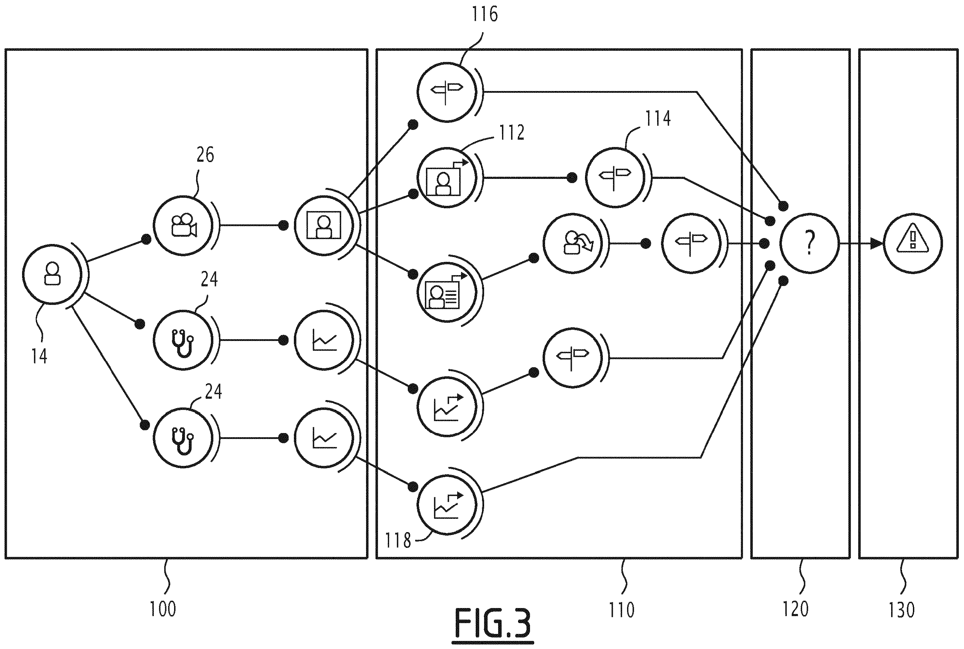

Le fonctionnement du dispositif électronique de surveillance 22 selon l'invention va désormais être expliqué à l'aide de la figure 3 représentant un organigramme du procédé, selon l'invention, de surveillance d'un état de conscience d'un opérateur 14 dans un poste de commande 16 d'un aéronef 12.

Initialement, l'aéronef 12 est en situation opérationnelle de vol, volant par exemple en destination d'un aéroport.

Au moins un opérateur 14 est présent dans le poste de commande 16 de l'aéronef 12. L'opérateur 14 est par exemple un pilote, comme illustré ici.

Le poste de commande 16 comprend au moins deux capteurs, dont au moins l'un des capteurs est un capteur porté 24 par l'opérateur 14 et au moins l'un des capteurs est un capteur déporté 26, à distance de l'opérateur.

Le procédé comprend une étape initiale 100 de réception par le module de réception 30 d'au moins une donnée du capteur porté 24 et au moins une donnée du capteur déporté 26.

A titre d'exemple, le capteur porté 24 est un capteur cardiaque et la donnée mesurée sont les battements du coeur au cours du temps. Le capteur déporté 26 est par exemple une caméra placée en vis-à-vis de l'opérateur 14 dans le poste de commande 16 et la donnée associée est une succession d'images au cours du temps du buste de l'opérateur 14.

Ces données étant brutes, elles sont avantageusement traitées. Les données sont notamment normalisées et centrées. Le prétraitement comprend en outre la vérification de l'échantillonnage des données, la présence d'artefacts ou de bruit capteur. Le prétraitement peut comprendre également un filtrage pour enlever la tendance des signaux, i.e. des éléments basse fréquence non pertinents pour la surveillance.

Puis, le procédé comprend une étape 110 d'extraction par le module de traitement 32 de chaque donnée d'au moins un paramètre représentatif de l'état de conscience de l'opérateur 14.

En particulier, le module de traitement 32 extrait de chaque donnée au moins un paramètre représentatif de l'état de conscience de l'opérateur 14 en effectuant :

- une extraction 112 d'une caractéristique prédéterminée de la donnée associée suivi de la mise en oeuvre 114 d'une méthode d'apprentissage automatique ;
- la mise en oeuvre 116 d'une méthode d'apprentissage profond appliquée directement sur la donnée associée ; ou
- une modélisation 118 prédéterminée appliquée à la donnée associée.

Toujours dans le même exemple, le module de traitement 32 extrait de la donnée du capteur porté 24 un rythme cardiaque comparé à un rythme cardiaque de repos prédéterminé. Le module de traitement 32 extrait de la donnée du capteur déporté 26 par exemple une fréquence et une durée de clignement des yeux de l'opérateur 14 et/ou par exemple une position de la tête de l'opérateur 14.

Puis, le procédé comprend une étape 120 de réception par le module de fusion 34 des dits paramètres représentatifs et de mise en oeuvre d'une méthode d'apprentissage automatique pour déterminer en fonction des paramètres représentatifs si l'opérateur 14 est dans un état de conscience nominal ou dans un état de conscience altéré.

Toujours dans le même exemple, le module de fusion 34 reçoit le rythme cardiaque et la fréquence et une durée de clignement des yeux et en déduit l'état de conscience de l'opérateur 14. La fusion de ces deux paramètres permet une meilleure estimation. En effet, le rythme cardiaque de l'opérateur 14 peut être assez élevé et ne pas être associé à une perte de connaissance mais la durée importante des clignements des yeux traduit un état de conscience altéré de l'opérateur 14. A l'inverse, les yeux du pilote peuvent rester ouverts normalement mais un rythme cardiaque bas peut traduire une somnolence du pilote.

Avantageusement, le procédé comprend une étape 130 d'émission d'un signal d'alerte lorsque le module de fusion 34 détermine que l'opérateur est dans un état de conscience altéré.

On conçoit alors que la présente invention présente un certain nombre d'avantages.

En effet, l'utilisation de plusieurs capteurs de différents types et la fusion des paramètres représentatifs permet d'obtenir un dispositif de surveillance de l'état de conscience d'un opérateur permettant une meilleure réactivité tout en étant précis, fiable et robuste.

En particulier, le dispositif de surveillance selon l'invention est plus robuste aux variabilités dues à l'opérateur, à l'environnement ainsi qu'aux conditions de vol, tout en conservant une réactivité accrue à la détection d'un état de conscience dégradé de l'opérateur par la multiplication et la diversification des données collectées via les différents capteurs.

Enfin, l'invention permet une meilleure disponibilité de la détection d'une perte de conscience et permet de réduire le nombre de faux-positifs.

## Revendications

1. Dispositif électronique de surveillance (22) d'un état de conscience d'un opérateur (14) dans un poste de commande (16) d'un aéronef (12), le dispositif de surveillance (22) comprenant :

   - un module de réception (30) configuré pour recevoir une donnée d'au moins deux capteurs embarqués dans l'aéronef (12), au moins l'un des capteurs dit capteur porté (24) étant au contact physique dudit opérateur (14) et au moins l'un des capteurs dit capteur déporté (26) étant à distance dudit opérateur (14) ;
   - un module de traitement (32) configuré pour extraire de chaque donnée au moins un paramètre représentatif de l'état de conscience de l'opérateur (14) ;
   - un module de fusion (34) configuré pour recevoir les dits paramètres représentatifs et mettre en oeuvre une méthode d'apprentissage automatique pour déterminer en fonction des paramètres représentatifs si l'opérateur (14) est dans un état de conscience nominal ou dans un état de conscience altéré.

2. Dispositif de surveillance (22) selon la revendication 1, dans lequel le dispositif de surveillance (22) comprend en outre un module d'alerte (36) configuré pour émettre un signal d'alerte lorsque le module de fusion (34) détermine que l'opérateur (14) est dans un état de conscience altéré.

3. Dispositif de surveillance (22) selon la revendication 1 ou 2, dans lequel chaque capteur porté (24) est choisi parmi le groupe constitué de :

   - un capteur cardiaque, notamment un électrocardiographe ;
   - un oxymètre de pouls, notamment un capteur à photopléthysmographie ;
   - un capteur de respiration ;
   - un accéléromètre ;
   - une électrode crânienne, par exemple un électroencéphalographe ;
   - un capteur de pression agencé dans un siège (18) de l'opérateur (14) ;
   - un capteur de pression agencé dans un dispositif de commande (19) propre à être actionné par l'opérateur (14) ;
   - un capteur de sudation de l'opérateur (14) ;
   - un capteur de réponse électrodermale ;
   - un capteur de température interne de l'opérateur (14);
   - un bandeau à spectroscopie proche infrarouge.

4. Dispositif de surveillance (22) selon la revendication 3, dans lequel au moins l'un des capteurs portés (24) est un capteur de pression configuré pour mesurer au moins une pression appliquée par l'opérateur (14) sur le capteur de pression, le paramètre associé propre à être extrait par le module de traitement étant une durée durant laquelle la pression mesurée est supérieure à une pression seuil prédéterminée.

5. Dispositif de surveillance (22) selon la revendication 3 ou 4, dans lequel au moins l'un des capteurs portés (24) est un accéléromètre configuré pour mesurer une accélération d'au moins une partie de l'opérateur (14), le paramètre associé propre à être extrait par le module de traitement (32) étant une signature issue d'une analyse fréquentielle et/ou une analyse temporelle de l'accélération mesurée et choisi parmi le groupe constitué de :

   - une puissance portée par une bande de fréquence de l'accélération mesurée ;
   - un rapport entre puissances de bandes de fréquences de l'accélération mesurée ;
   - la puissance de l'accélération mesurée ;
   - la moyenne de l'accélération mesurée ;
   - le taux de passage par zéro de l'accélération mesurée ;

- la régularité de l'accélération mesurée ;
- la complexité de l'accélération mesurée ;
- l'entropie de l'accélération mesurée ;
- les paramètres d'une modélisation de l'accélération mesurée ;
- les coefficients issus d'une analyse temps fréquence de l'accélération mesurée ;
- les coefficients issus d'une analyse temps échelle de l'accélération mesurée.

6. Dispositif de surveillance (22) selon l'une quelconque des revendications précédentes, dans lequel chaque capteur déporté (26) est choisi parmi le groupe constitué de :

   - une caméra configurée pour capter au moins une image comprenant au moins une partie de l'opérateur (14) ;
   - un microphone pour capter au moins une son émis par l'opérateur (14), tel que sa voix ou sa respiration ;
   - un capteur infrarouge de température de peau de l'opérateur (14).

7. Dispositif de surveillance (22) selon la revendication 6, dans lequel au moins l'un des capteurs déportés (26) est une caméra configurée pour capter au moins une image comprenant au moins une partie de l'opérateur (14), chaque paramètre propre à être extrait par le module de traitement (32) étant choisi parmi le groupe constitué de :

   - un mouvement de l'opérateur (14) ;
   - une posture de l'opérateur (14) ;
   - une orientation de la tête de l'opérateur (14) ;
   - une direction du regard de l'opérateur (14) ;
   - une ouverture partielle des yeux de l'opérateur (14);
   - un clignement des yeux de l'opérateur (14);
   - une information de structure de l'image dans laquelle l'opérateur (14) apparaît.

8. Dispositif de surveillance (22) selon l'une quelconque des revendications précédentes, dans lequel au moins l'un des capteurs portés (24) est un capteur de pression, au moins l'un des capteurs portés (24) est un accéléromètre et au moins l'un des capteurs déporté (26) est une caméra.

9. Dispositif de surveillance (22) selon l'une quelconque des revendications précédentes, dans lequel le module de traitement (32) est configuré pour extraire de chaque donnée au moins un paramètre représentatif de l'état de conscience de l'opérateur (14) en mettant en oeuvre pour chaque donnée un algorithme choisi parmi le groupe constitué de:

   - une extraction d'une caractéristique prédéterminée de la donnée associée suivi d'une méthode d'apprentissage automatique ;
   - une méthode d'apprentissage profond appliquée directement sur la donnée associée ;
   - une modélisation prédéterminée appliquée à la donnée associée.

10. Procédé de surveillance d'un état de conscience d'un opérateur (14) dans un poste de commande (16) d'un aéronef (12), le procédé de surveillance comprenant au moins les étapes suivantes :

    - réception (100) d'une donnée d'au moins deux capteurs embarqués dans l'aéronef (12), au moins l'un des capteurs dit capteur porté (24) étant au contact physique dudit opérateur (14) et au moins l'un des capteurs dit capteur déporté (26) étant à distance dudit opérateur (14) ;
    - extraction (110) de chaque donnée d'au moins un paramètre représentatif de l'état de conscience de l'opérateur (14) ;
    - réception (120) des dits paramètres représentatifs et mise en oeuvre d'une méthode d'apprentissage automatique pour déterminer en fonction des paramètres représentatifs si l'opérateur (14) est dans un état de conscience nominal ou dans un état de conscience altéré.

11. Programme d'ordinateur comportant des instructions logicielles qui, lorsqu'elles sont exécutées par un ordinateur, mettent en oeuvre un procédé de surveillance selon la revendication précédente.

**FIG.1**

**FIG.2**

FIG.3

Europäisches
Patentamt

European
Patent Office

Office européen
des brevets

# RAPPORT DE RECHERCHE EUROPEENNE

Numéro de la demande

**EP 23 16 8960**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 2021/122900 A1 (THALES SA [FR]) 24 juin 2021 (2021-06-24) | 1-3,5-11 | INV. G08B21/06 |
| Y | * abrégé; figures 1-6 * <br> * [0002], [0017], [0023], [0029]-[0032], [0042]-[0045], [0051], [0053], [0059], [0070], [0071] * | 4 | A61B5/00 B60K28/00 |
| | ----- | | |
| Y | CN 110 942 592 A (CHANGAN UNIV) 31 mars 2020 (2020-03-31) * [0025] de la traduction automatique * | 4 | |
| | ----- | | |

**DOMAINES TECHNIQUES RECHERCHES (IPC)**

G08B
B60K
G06K
A61B

Le présent rapport a été établi pour toutes les revendications

| Lieu de la recherche | Date d'achèvement de la recherche | Examinateur |
|---|---|---|
| Munich | 25 août 2023 | Bilard, Stéphane |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**EP 4 266 281 A1**

## ANNEXE AU RAPPORT DE RECHERCHE EUROPEENNE
## RELATIF A LA DEMANDE DE BREVET EUROPEEN NO.

EP 23 16 8960

25-08-2023

| Document brevet cité au rapport de recherche | Date de publication | Membre(s) de la famille de brevet(s) | Date de publication |
|---|---|---|---|
| WO 2021122900 A1 | 24-06-2021 | AU 2020407832 A1 | 28-07-2022 |
| | | EP 4078609 A1 | 26-10-2022 |
| | | FR 3104935 A1 | 25-06-2021 |
| | | WO 2021122900 A1 | 24-06-2021 |
| CN 110942592 A | 31-03-2020 | AUCUN | |

EPO FORM P0460